# EUROPEAN PATENT APPLICATION

(11) **EP 4 292 523 A1**
(43) Date of publication of application: **20.12.2023**
(21) Application number: 23175875.6
(22) Date of filing: 27.05.2023
(51) Int. Cl.: A61B 5/0205, A61B 5/024, A61B 5/08, A61B 5/11

(54) **PHYSIOLOGICAL PARAMETER MONITORING SYSTEM AND METHOD, STORAGE MEDIUM**

(30) Priority: 14.06.2022 CN 202210669846
(71) Applicant: Shenzhen Mindray Bio-Medical Electronics Co., Ltd., Shenzhen, Guangdong 518057 (CN)
(72) Inventor: XIAO, Ke, Shenzhen, 518057 (CN); JIN, Xingliang, Shenzhen, 518057 (CN); HE, Xianliang, Shenzhen, 518057 (CN); WANG, Rendong, Shenzhen, 518057 (CN); MENG, Libin, Shenzhen, 518057 (CN)
(74) Representative: KIPA AB

(57) **Abstract**

Provided are a physiological parameter monitoring system and method, and as well as a storage medium. The monitoring system includes a first measurement unit, a second measurement unit and a processor. On the one hand, one measurement unit is used to mainly measure vital signs and another one measurement unit is used to mainly measure movements, so that interference can be comprehensively determined through information correction to avoid inaccurate measurement of vital signs caused by a single measurement unit. On the other hand, adding a second measurement unit can overcome the problem of poor anti-interference performance when using radar device alone for non-contact vital sign measurement. By selecting, fusing, or correcting the information measured by different measurement units and participating in the processing process of vital sign information, the reliability of the system can be enhanced, such that more accurate vital sign measurement results can be obtained.

## Description

### TECHNICAL FIELD

The disclosure relates to a physiological parameter monitoring system and method, and as well as a storage medium, for measuring various physiological parameters of a subject, including heartbeat, respiration, etc., through a non-contact method.

### BACKGROUND

In existing physiological information measurement technologies, contact measurement methods are often used. For example, when measuring heartbeat information, electrocardiogram pads need to be attached to different body parts of the measured object, or when measuring respiratory information through an acoustic measurement method, acoustic sensors need to be attached to body surface or outer side of a respiratory tract of the measured object.

There are many shortcomings in various contact measurement methods, such as in the monitoring of newborns (premature infants), where skin development of premature infant is incomplete, and sensors attached to the skin surface may cause skin damage in newborns, leading to fatal infections. The same applies to adult patients who are prone to skin damage. Furthermore, the sensors used for contact measurement are usually sterile consumable, which increases the cost of patient monitoring and subsequently increases the medical expense of patient. In addition, each sensor for contact measurement can only measure one individual and require the use of connection wires to connect the sensor to a back-end processing system. The wired measurement method to some extent increases the burden of medical care.

In response to the limitations of contact measurement methods, some have considered using non-contact methods to measure physiological information. However, due to factors, such as non-target movement interference, non-contact measurement methods still have shortcomings in reliability and anti-interference ability, and further technical improvements are needed.

### SUMMARY

This disclosure mainly provides a physiological parameter monitoring system and method, and as well as a storage medium, aiming at shortcomings of existing contact and non-contact methods for measuring physiological information.

According to a first aspect, an embodiment of this disclosure provides a physiological parameter monitoring system, including: a first measurement unit, which is configured to implement non-contact measurement on a measured object to acquire a first measurement signal, wherein the first measurement signal includes at least first vital sign information of the measured object; a second measurement unit, which is configured to implement non-contact measurement on the measured object to acquire a second measurement signal, wherein the second measurement signal at least includes second movement information which is caused by a movement that affects a vital sign of the measured object; and a processor which is configured to acquire the first measurement signal of the first measurement unit and extract the first vital sign information from the first measurement signal, to acquire the second measurement signal of the second measurement unit and extract the second movement information from the second measurement signal, and to at least correct the first vital sign information according to the second movement information, so as to obtain a physiological parameter of the measured object.

In an embodiment, the first measurement signal further includes first movement information which is caused by the movement that affects the vital sign of the measured object, and the processor is configured to extract the first movement information from the first measurement signal; wherein in order to correct the first vital sign information according to the second movement information, so as to obtain a physiological parameter of the measured object, the processor is further configured to correct the first vital sign information, at least according to the first movement information and the second movement information, so as to obtain the physiological parameter of the measured object.

In an embodiment, in order to correct the first vital sign information, at least according to the first movement information and the second movement information, so as to obtain the physiological parameter of the measured object, the processor is further configured to: process the first movement information and the second movement information to obtain final movement information; and correct the first vital sign information by using the final movement information, so as to obtain the physiological parameter of the measured object.

In an embodiment, in order to correct the first vital sign information, at least according to the first movement information and the second movement information, so as to obtain the physiological parameter of the measured object, the processor is further configured to: correct the first vital sign information, by using one of the first movement information and the second movement information, to obtain first vital sign data; and correct the first vital sign data, by using the other one of the first movement information and the second movement information, so as to obtain the physiological parameter of the measured object.

In an embodiment, in order to process the first movement information and the second movement information to obtain final movement information, the processor is further configured to perform an information weighted fusion calculation on the first movement information and the second movement information to obtain the final movement information.

In an embodiment, the correction includes correcting a magnitude of a value or maintaining a value unchanged for a predetermined time.

In an embodiment, the first vital sign information includes one or more of respiration, heartbeat, pulse, blood pressure, and body temperature; wherein each of the first movement information and the second movement information includes one or more of a limb movement of the measured object, a limb movement of a nurse, and a movement of an obstacle.

According to a second aspect, an embodiment of this disclosure provides a physiological parameter monitoring system, including: a first measurement unit, which is configured to implement non-contact measurement on a measured object to acquire a first measurement signal, wherein the first measurement signal includes first vital sign information of the measured object and first movement information which is caused by a movement that affects a vital sign of the measured object; a second measurement unit, which is configured to implement non-contact measurement on the measured object to acquire a second measurement signal, wherein the second measurement signal includes second vital sign information of the measured object and second movement information which is caused by the movement that affects the vital sign of the measured object; and a processor, which is configured to acquire the first measurement signal of the first measurement unit and extract the first vital sign information and the first movement information, to acquire the second measurement signal of the second measurement unit and extract the second vital sign information and the second movement information from the second measurement signal; wherein the processor is further configured to correct at least one of the first vital sign information and the second vital sign information, according to at least one of the first movement information and the second movement information, so as to obtain a physiological parameter of the measured object.

In an embodiment, in order to correct at least one of the first vital sign information and the second vital sign information, according to at least one of the first movement information and the second movement information, so as to obtain a physiological parameter of the measured object, the processor is further configured to: analyze the first vital sign information and the second vital sign information to obtain second vital sign data through an information quantification comparison; process the first movement information and the second movement information to obtain final movement information; and correct the second vital sign data by using the final movement information, so as to obtain the physiological parameter of the measured object.

In an embodiment, in order to correct at least one of the first vital sign information and the second vital sign information, according to at least one of the first movement information and the second movement information, so as to obtain a physiological parameter of the measured object, the processor is further configured to: analyze the first vital sign information and the second vital sign information to obtain second vital sign data through an information quantification comparison; correct the second vital sign data, by using one of the first movement information and the second movement information, to obtain third vital sign data; correct the third vital sign data, by using the other one of the first movement information and the second movement information, to obtain fourth vital sign data; and obtain the physiological parameter of the measured object according to the third vital sign data or the fourth vital sign data.

In an embodiment, in order to correct at least one of the first vital sign information and the second vital sign information, according to at least one of the first movement information and the second movement information, so as to obtain a physiological parameter of the measured object, the processor is further configured to: correct the first vital sign information by using the first movement information to obtain fifth vital sign data; correct the second vital sign information by using the second movement information to obtain sixth vital sign data; and analyze the fifth vital sign data and the sixth vital sign data, so as to obtain the physiological parameter of the measured object through a numerical quantification comparison.

In an embodiment, in order to correct at least one of the first vital sign information and the second vital sign information, according to at least one of the first movement information and the second movement information, so as to obtain a physiological parameter of the measured object, the processor is further configured to: correct one of the first vital sign information and the second vital sign information, by using one of the first movement information and the second movement information, to obtain seventh vital sign data; analyze uncorrected vital sign information in the first vital sign information and the second vital sign information with the seventh vital sign data, so as to obtain the physiological parameter of the measured object through a numerical quantification comparison.

In an embodiment, in order to process the first movement information and the second movement information to obtain final movement information, the processor is further configured to: calculate a respective information confidence level which corresponds to the first movement information and the second movement information, and take the movement information, which corresponds to a higher information confidence level in the first movement information and the second movement information, as the final movement information; or perform an information weighted fusion calculation on the first movement information and the second movement information to obtain the final movement information.

In an embodiment, the correction includes correcting a magnitude of a value or maintaining a value unchanged for a predetermined time.

In an embodiment, in order to analyze the first vital sign information and the second vital sign information to obtain second vital sign data through an information quantification comparison, the processor is further configured to: calculate a respective information confidence level which corresponds to the first vital sign information and the second vital sign information, and take the vital sign information, which corresponds to a higher information confidence level in the first vital sign information and the second vital sign information, as the second vital sign data; or perform an information weighted fusion calculation on the first vital sign information and the second vital sign information to obtain the second vital sign data.

In an embodiment, in order to analyze the fifth vital sign data and the sixth vital sign data, so as to obtain the physiological parameter of the measured object through a numerical quantification comparison, the processor is further configured to: normalize the first movement information and the second movement information respectively to obtain a corresponding interference amplitude, and take the vital sign data with a smaller interference amplitude in the fifth vital sign data and the sixth vital sign data as the physiological parameter of the measured object; or calculate a respective information confidence level which corresponds to the first vital sign information and the second vital sign information, and take the vital sign data, which corresponds to a higher information confidence level in the fifth vital sign data and the sixth vital sign data, as the physiological parameter of the measured object; or perform an information weighted fusion calculation on the fifth vital sign data and the sixth vital sign data, so as to obtain the physiological parameter of the measured object.

In an embodiment, each of the first vital sign information and the second vital sign information includes one or more of respiration, heartbeat, pulse, blood pressure, and body temperature; each of the first movement information and the second movement information includes one or more of a limb movement of the measured object, a limb movement of a nurse, and a movement of an obstacle.

In an embodiment, the first measurement unit includes a radar device; the second measurement unit includes one or more of: a radar device, an optical camera, an infrared sensor, an infrared thermal imager, a depth measurement sensor, a sound sensor, a pressure sensor, and an acceleration sensor.

According to a third aspect, an embodiment of this disclosure provides a physiological parameter monitoring method, including extracting first vital sign information of a measured object from a measurement signal of one measurement unit; extracting second movement information, which is caused by a movement that affects a vital sign of the measured object, from a measurement signal of another measurement unit; and at least correcting the first vital sign information according to the second movement information, so as to obtain a physiological parameter of the measured object.

In an embodiment, the physiological parameter monitoring method further includes: extracting first movement information which is caused by the movement that affects the vital sign of the measured object, when extracting the first vital sign information of the measured object from the measurement signal of the one measurement unit; and at least correcting the first vital sign information, according to the first movement information and the second movement information, so as to obtain the physiological parameter of the measured object.

In an embodiment, at least correcting the first vital sign information, according to the first movement information and the second movement information, so as to obtain the physiological parameter of the measured object, includes: processing the first movement information and the second movement information to obtain final movement information; and correcting the first vital sign information by using the final movement information, so as to obtain the physiological parameter of the measured object.

In an embodiment, at least correcting the first vital sign information, according to the first movement information and the second movement information, so as to obtain the physiological parameter of the measured object, includes: correcting the first vital sign information, by using one of the first movement information and the second movement information, to obtain first vital sign data; and correcting the first vital sign data, by using the other one of the first movement information and the second movement information, so as to obtain the physiological parameter of the measured object.

According to a fourth aspect, an embodiment of this disclosure provides a physiological parameter monitoring method, including extracting, from a measurement signal of one measurement unit, first vital sign information of a measured object and first movement information, which is caused by an movement that affects a vital sign of the measured object; extracting, from a measurement signal of another measurement unit, second vital sign information of the measured object and second movement information, which is caused by the movement that affects the vital sign of the measured object; and correcting at least one of the first vital sign information and the second vital sign information, according to at least one of the first movement information and the second movement information, so as to obtain a physiological parameter of the measured object.

In an embodiment, correcting at least one of the first vital sign information and the second vital sign information, according to at least one of the first movement information and the second movement information, so as to obtain a physiological parameter of the measured object, includes: analyzing the first vital sign information and the second vital sign information to obtain second vital sign data through an information quantification comparison; processing the first movement information and the second movement information to obtain final movement information; and correcting the second vital sign data by using the final movement information, so as to obtain the physiological parameter of the measured object.

In an embodiment, correcting at least one of the first vital sign information and the second vital sign information, according to at least one of the first movement information and the second movement information, so as to obtain a physiological parameter of the measured object, includes: analyzing the first vital sign information and the second vital sign information to obtain second vital sign data through an information quantification comparison; correcting the second vital sign data, by using one of the first movement information and the second movement information, to obtain third vital sign data; correcting the third vital sign data, by using the other one of the first movement information and the second movement information, to obtain fourth vital sign data; and obtaining the physiological parameter of the measured object according to the third vital sign data or the fourth vital sign data.

In an embodiment, correcting at least one of the first vital sign information and the second vital sign information, according to at least one of the first movement information and the second movement information, so as to obtain a physiological parameter of the measured object, includes: correcting the first vital sign information by using the first movement information to obtain fifth vital sign data; correcting the second vital sign information by using the second movement information to obtain sixth vital sign data; and analyzing the fifth vital sign data and the sixth vital sign data through a numerical quantification comparison, so as to obtain the physiological parameter of the measured object.

In an embodiment, correcting at least one of the first vital sign information and the second vital sign information, according to at least one of the first movement information and the second movement information, so as to obtain a physiological parameter of the measured object, includes: correcting one of the first vital sign information and the second vital sign information, by using one of the first movement information and the second movement information, to obtain seventh vital sign data; analyzing uncorrected vital sign information in the first vital sign information and the second vital sign information with the seventh vital sign data, so as to obtain the physiological parameter of the measured object through a numerical quantification comparison.

In a fifth aspect, a computer-readable storage medium on which a computer program is stored, is provided, wherein when the computer program is executed by a processor, steps of the physiological parameter monitoring method described according to the third and fourth aspects, are implemented.

The beneficial effects of this disclosure are as follows. The above embodiments recite a physiological parameter monitoring system and method, and as well as a storage medium, wherein the physiological parameter monitoring system includes a first measurement unit, a second measurement unit and a processor. On the one hand, the above embodiments use one measurement unit to mainly measure vital signs and another one measurement unit to mainly measure movements, so that interference can be comprehensively determined through information correction to avoid inaccurate measurement of vital signs caused by a single measurement unit. On the other hand, adding a second measurement unit can overcome the problem of poor anti-interference performance when using radar device alone for non-contact vital sign measurement. By selecting, fusing, or correcting the information measured by different measurement units and participating in the processing process of vital sign information, the reliability of the system can be enhanced, such that more accurate vital sign measurement results can be obtained.

### BRIEF DESCRIPTION OF THE DRAWINGS

FIG. 1 is a structural diagram of a physiological parameter monitoring system in one embodiment of this disclosure.
FIG. 2 is a schematic diagram of using a physiological parameter monitoring system in one embodiment of this disclosure to monitor new-borns.
FIG. 3 is a schematic diagram of information processing process in one embodiment of this disclosure.
FIG. 4 is a schematic diagram of a first information processing situation in one embodiment of this disclosure.
FIG. 5 is a schematic diagram of a second information processing situation in one embodiment of this disclosure.
FIG. 6 is a schematic diagram of information processing process in another embodiment of this disclosure.
FIG. 7 is a schematic diagram of a first information processing situation in another embodiment of this disclosure.
FIG. 8 is a schematic diagram of a second information processing situation in another embodiment of this disclosure.
FIG. 9 is a schematic diagram of a third information processing situation in another embodiment of this disclosure.
FIG. 10 is a schematic diagram of a fourth information processing situation in another embodiment of this disclosure.
FIG. 11 is a flowchart of a physiological parameter monitoring method in an embodiment of this disclosure.
FIG. 12 is a flowchart of a physiological parameter monitoring method in another embodiment of this disclosure.
FIG. 13 is a flowchart of a physiological parameter monitoring method in another further embodiment of this disclosure.
FIG. 14 is a structural diagram of an information processing device in an embodiment of this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, so as to avoid the core part of this disclosure being inundated by too many descriptions. For one skilled in the art, it is not necessary to describe these related operations in detail, as they can fully understand the related operations according to the description in this disclosure and the general technical knowledge in the art.

In addition, the features, operations, or characteristics described in the specification may be combined in any appropriate manner to form various embodiments. At the same time, the steps or movements in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are used to distinguish different objects, rather than to describe a specific order. In addition, the terms "connection", and "linkage" mentioned in this disclosure include direct and indirect connection (linkage), unless otherwise specified.

In the scenario of non-contact measurement of physiological parameter of the measured object, using radar, such as UWB and FMCW, to measure the physiological parameter is an important physiological parameter monitoring method. This method achieves physiological parameter monitoring through remotely and non-contact measuring movement (vibration) information generated by physiological movements such as heartbeat, respiration, and pulse pulsation of the measured object. Due to the relatively weak amplitude of such physiological movements, measurement system that solely uses radar is highly susceptible to interference from non-target movements, such as the limb or head movements of the measured object, or the movements of other individuals or objects within the radar detection range. The interference signal amplitude introduced by non-target movement is difficult to be separated from the physiological signal to be measured, and the interference signal amplitude is usually greater than the amplitude of the physiological signal to be measured. This not only submerges the original physiological signal, but also may form a pseudo physiological signal, causing the monitoring system to mistakenly determine the physiological state of the measured object, reducing the reliability of the system and delaying determination for the physiological state of the patient by the medical staff. Therefore, in order to improve the reliability and anti-interference performance of non-contact measurement of physiological parameter, this disclosure introduces two non-contact measurement units, which jointly identify interference movements and accurately calculate the physiological parameters of the measured object through multiple measurement units.

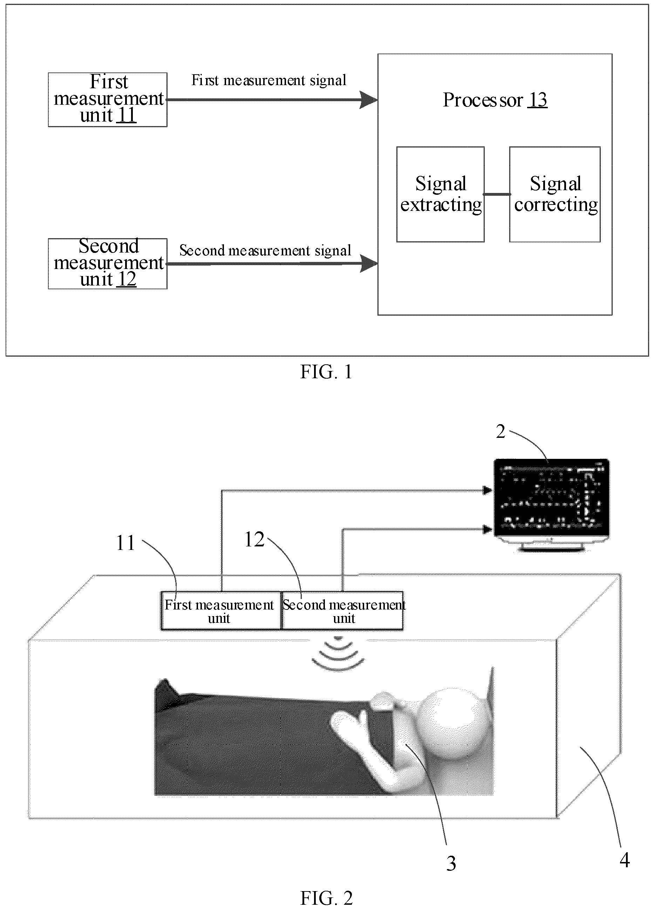

Please refer to FIG. 1, a physiological parameter monitoring system is disclosed, which mainly includes a first measurement unit 11, a second measurement unit 12, and a processor 13, as explained below.

Among them, the first measurement unit 11 is arranged around the measured object (such as patients, newborns, etc.), and configured to perform non-contact measurement on the measured object to acquire a first measurement signal. The main purpose of arranging the first measurement unit 11 is to understand vital signs of the measured object, so that the first measurement signal can at least contain first vital sign information of the measured object.

Among them, the second measurement unit 12 is also arranged around the measured object, and configured to perform non-contact measurement on the measured object to obtain a second measurement signal. The main purpose of arranging the second measurement unit is to understand an interference movement around the measured object, so that the second measurement signal can at least contain second movement information which is caused by a movement that affects a vital sign of the measured object.

Among them, the processor 13 is in signal connection with both the first measurement unit 11 and the second measurement unit 12. The processor 13 can not only acquire the first measurement signal of the first measurement unit 11 and extract the first vital sign information from the first measurement signal, but also can acquire the second measurement signal of the second measurement unit 12 and extract the second movement information from the second measurement signal. In addition, processor 13 also has information processing capability, which can correct at least the first vital sign information according to the second movement information, thereby obtaining a physiological parameter of the measured object after processing.

For example, FIG. 2 illustrates using a physiological parameter monitoring system in one embodiment of this disclosure to monitor new-borns. For newborn 3 (or premature infant), which is a measured object, it is often necessary to be placed in an incubator 4 for a real-time monitoring. Due to the incomplete skin development of newborn 3, sensors cannot be attached to his/her skin surface, which requires to use the first measurement unit 11 and the second measurement unit 12 installed at the incubator 4 to conduct non-contact measurements on newborn 3 for understanding vital sign state of newborn 3 in real-time. Meanwhile, both the first measurement unit 11 and the second measurement unit 12 are connected with a monitoring device 2 (such as a life monitor). Of course, the monitoring device 2 should have a processor 13 with signal analysis and information processing capabilities, and the processor 13 should be able to receive the first measurement signal from the first measurement unit 11 and the second measurement signal from the second measurement unit 12, respectively.

It should be noted that the first measurement unit 11 and the second measurement unit 12 can be the same measurement device or different measurement device. In one embodiment, measurement principles of the two are different, and even if their measurement principles are the same, they can have different measurement concerns. Preferably, the first measurement unit 11 includes a radar device, while the second measurement unit 12 includes one or more of: a radar device, an optical camera, an infrared sensor, an infrared thermal imager, a depth measurement sensor, a sound sensor, a pressure sensor, and an acceleration sensor. It can be understood that the radar device can transmit and receive a radar signal in the form of radio wave, and through radar detection, the vital sign of the measured object and the movement that constitute measurement interference can be obtained. The optical camera can continuously capture and form optical images, and record changes in the vital signs of the measured object and changes in the movements that constitute interference through frames of optical images. The infrared sensor can transmit and receive infrared light, and acquire the vital signs of the measured object and the movements that constitute measurement interference through infrared detection. The infrared thermal imager can continuously capture and form infrared images, and record the changes in the vital signs of the measured object and the changes in the movements that constitute interference through frames of infrared images. The depth measurement sensor can measure field depth of all objects in a view field, and represent the changes in the vital signs of the measured object and the changes in the movements that constitute interference through changes in depth of field. The sound sensor can be distributed in an array to detect slight sounds from multiple directions, thus understanding the changes in the vital signs of the measured object and the changes in the movements that constitute interference through sound changes. The pressure sensor can be arranged in an array and installed at clothing or back pad of the measured object, and the pressure sensor can detect pressure in multiple directions thus understanding the changes in the vital signs of the measured object and the changes in the movements that constitute interference through pressure changes. The acceleration sensor can be arranged in an array and installed at clothing or back pad of the measured object and the acceleration sensor can detect movement in multiple directions, thus understanding the changes in the vital signs of the measured object and the changes in the movements that constitute interference through movement changes. Of course, the first measurement unit 11 mainly uses radar device because radar detection technology is more mature and effective, so it can be used as the main device for measuring vital signs. Although the second measurement unit 12 can use radar device or other sensor devices, it can be viewed as an auxiliary device to measure interference factors and correct vital sign parameters. If both the first measurement unit 11 and the second measurement unit 12 use radar device, the radar device can also transmit and receive radar signals of different bands, thereby enhancing the measurement effectiveness of specific information.

It should be noted that the first measurement signal generated by the first measurement unit 11 through measurement can be an analog signal or digital signal that follows a certain transmission protocol, while the second measurement signal generated by the second measurement unit 12 through measurement can also be an analog signal or digital signal that follows a certain transmission protocol. Due to the ability of both analog and digital signals to stipulate useful information, the first vital sign information contained in the first measurement signal includes one or more of respiration, heartbeat, pulse, blood pressure, and body temperature, all of which can reflect the vital sign state of the measured object. For example, the vital sign information taking respiration as an example includes but is not limited to a respiratory signal (respiratory wave), respiratory rate, respiratory amplitude, inhalation or exhalation state, whether apnea occurs, whether suffocation occurs, and number of occurrence times of apnea or suffocation per unit time. Due to the adverse effects of limb movements, wearing movements of the measured object, and rolling movements on the measured object of nurse on vital sign measurement, the second measurement signal includes one or more of a limb movement of the measured object, a limb movement of a nurse, and a movement of an obstacle. For example, taking the limb movement of the measured object as an example, the movement information includes but is not limited to whether interference movement occurs, magnitude of interference, and a duration of interference, etc.

It should be noted that the signal extracting ability of the processor 13, such as signal conditioning, signal analog-to-digital conversion, communication protocol conversion, and numerical quantization, can be achieved through an internal signal processing circuit. In this way, the processor 13 can extract the first vital sign information from the first measurement signal of the first measurement unit 11 and the second movement information from the second measurement signal of the second measurement unit 12. It can be understood that the first vital sign information and the second movement information can be digitized numerical quantification results for the convenience of subsequent information correction processing.

In FIG.1, the processor 13 can be devices such as CPU, MCU, FPGA, microcontroller, etc. Its information processing ability can be achieved through internal operational logic circuits. Therefore, at least the first vital sign information can be corrected according to the second movement information, so as to obtain the physiological parameter of the measured object. It can be understood that the physiological parameters of the measured object are quantified numerical results or fluctuation curves, which can correspond to the content of the first vital sign information, including one or more values of respiration, heartbeat, pulse, blood pressure, and body temperature. This facilitates users to directly understand the vital sign state of the measured object. The following provides a specific explanation of functions of the processor 13.

In one embodiment, as shown in FIG. 1, the processor 13 only corrects the first vital sign information in the first measurement signal according to the second movement information in the second measurement signal. The specific correction process can be divided into two optional situations. In optional situation (1), the processor 13 adjusts a numerical value of the first vital sign information by using the second movement information, such as subtracting the second movement information (or a product of the second movement information and an influence factor) from the first vital sign information and taking the obtained quantitative information as the physiological parameter of the measured object. In optional situation (2), the processor 13 utilizes the second movement information to maintain the first vital sign information at a fixed value for a preset time period, and if the second movement information is greater than a preset movement threshold, it indicates that the interference effect is severe, and the first vital sign information can be maintained at the previous value for a following preset time period (such as within 10 seconds) to avoid the occurrence of obvious inaccuracy of the vital sign information caused by the interference effect.

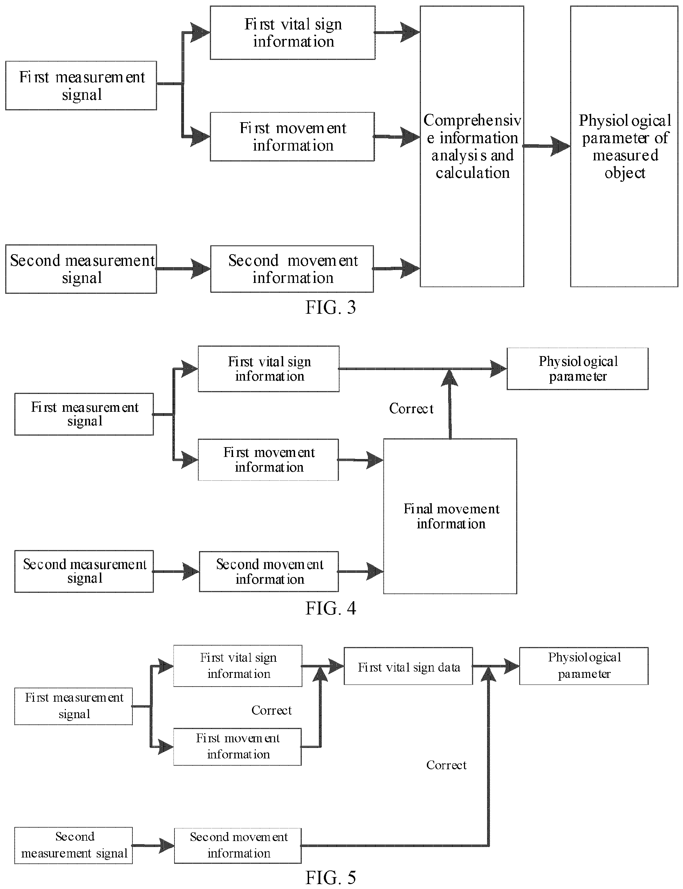

It can be understood that in the embodiment, one measurement unit is configured to mainly measure the vital sign, and another measurement unit is configured to mainly measure the movement. Separate measurements can not only strengthen multiple ways of information sources, but also comprehensively determine interference situations through information correction, thereby avoiding inaccurate measurement of the vital sign caused by a single measurement unit, and further providing better monitoring service for the measured object. In one embodiment, referring to FIGS 1 and 3, the first measurement signal of the first measurement unit 11 not only includes the first vital sign information, but also includes first movement information which is caused by a movement that affect the vital sign of the measured object, and the first movement information includes one or more of a limb movement of the measured object, a limb movement of a nurse, and a movement of an obstacle. For example, when taking the limb movement of the measured object as an example, the movement information includes but is not limited to whether interference movement occurs, magnitude of interference, and a duration of interference, etc. Accordingly, after receiving the first measurement signal from the first measurement unit 11, the processor 13 not only extracts the first vital sign information, but also extracts the first movement information from it. Moreover, the processor 13 extracts the second movement information from the second measurement signal of the second measurement unit 12. Afterwards, the processor 13 conducts comprehensive information analysis and calculation according to the first vital sign information, the first movement information, and the second movement information, such as correcting the first vital sign information, by using the first movement information and the second movement information, so as to obtain the physiological parameter of the measured object.

In a specific embodiment, refer to FIGS. 1, 3, and 4, an information processing method, that enables the processor 13 to correct the first vital sign information according to the first movement information and the second movement information, is provided. Specifically, the processor 13 processes the first movement information and the second movement information to obtain the final movement information. Then, processor 13 corrects the first vital sign information, by using the final movement information, so as to obtain the physiological parameter of the measured object.

Among them, the process of obtaining the final movement information can be divided into two optional situations. In optional situation (1), the processor 13 calculates an information confidence level, which corresponds to the first movement information and the second movement information, respectively, such as calculating an information confidence level of the first movement information according to an amplitude, frequency, and time-varying characteristic of a radar waveform signal measured by a radar device, and calculating an information confidence level of the second movement information according to an amplitude, frequency, and time-varying characteristic of an optical image signal measured by an optical camera, and takes the movement information which corresponds to a higher information confidence level in the first movement information and the second movement information as the final movement information. In optional situation (2), the processor 13 performs an information weighted fusion calculation on the first movement information and the second movement information, such as multiplying the first movement information with its corresponding weighting coefficient, multiplying the second movement information with its corresponding weighting coefficient and adding the products, to obtain the final movement information.

Among them, the process of obtaining the physiological parameter through the information correction can be divided into two optional situations. In optional situation (1), the processor 13 adjusts a numerical value of the first vital sign information by using the final movement information, such as subtracting the final movement information (or a product of the final movement information and an influence factor) from the first vital sign information and taking the obtained quantitative information as the physiological parameter of the measured object. In optional situation (2), the processor 13 utilizes the final movement information to maintain the first vital sign information at a fixed value for a preset time period, and if the final movement information is greater than a preset movement threshold, it indicates that the interference effect is severe, and the first vital sign information can be maintained at the previous value for a following preset time period (such as within 10 seconds) to avoid the occurrence of obvious inaccuracy of the vital sign information caused by the interference effect.

It should be noted that the correction of the first vital sign information mentioned earlier includes correcting a magnitude of a value or maintaining a value unchanged for a predetermined time.

In a specific embodiment, please refer to FIGS. 1, 3, and 5, another information processing method, that enables the processor 13 to correct the first vital sign information by using the first movement information and the second movement information, is provided. Specifically, the processor 13 corrects the first vital sign information to obtain first vital sign data by using one of the first movement information and the second movement information, and corrects the first vital sign data, by using the other one of the first movement information and the second movement information, so as to obtain the physiological parameter of the measured object.

The process of obtaining the first vital sign data through information correction can be divided into two optional situations. In optional situation (1), the processor 13 adjusts a numerical value of the first vital sign information according to the first movement information (or the second movement information) to obtain the first vital sign data. In optional situation (2), the processor 13 maintains a numerical value of the first vital sign information at a fixed value for a predetermined time period according to the first movement information (or the second movement information) to obtain the first vital sign data.

The process of obtaining the physiological parameter through information correction can be divided into two optional situations. In optional situation (1), the processor 13 adjusts a numerical value of the first vital sign data according to the second movement information (or the first movement information) to obtain the physiological parameter of the measured object. In optional situation (2), the processor 13 maintains the first vital sign data at a fixed value for a predetermined time period according to the second movement information (or the first movement information), so as to obtain the physiological parameter of the measured object.

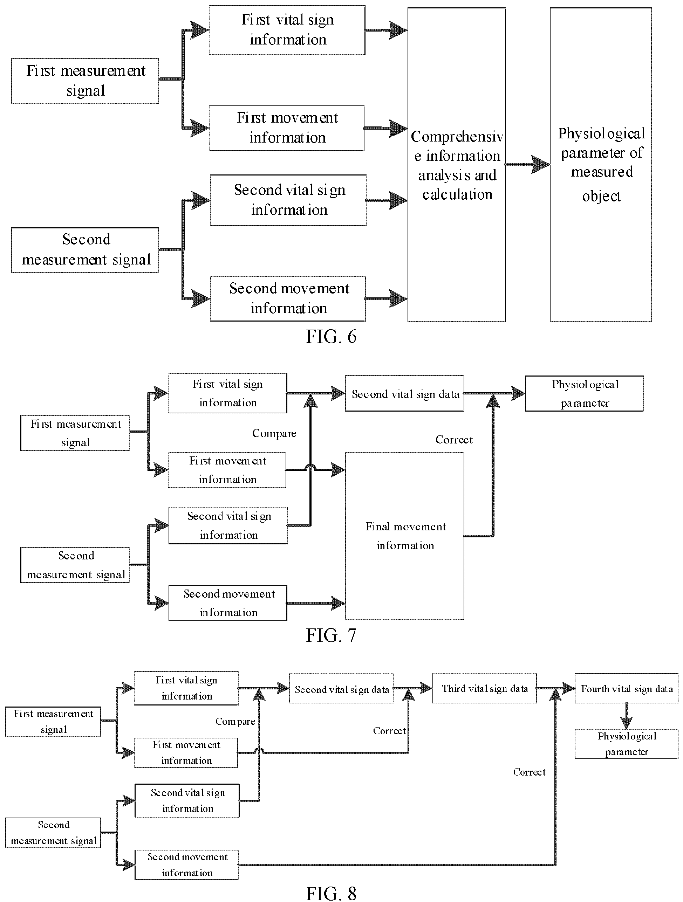

In one embodiment, as shown in FIGS. 1 and 6, the first measurement signal of the first measurement unit 11 not only includes first vital sign information, but also includes the first movement information which is caused by a movement that affects the vital sign of the measured object. The second measurement signal of the second measurement unit 12 not only includes the second movement information caused by a movement that affects the vital sign of the measured object, but also includes the second vital sign information. It can be understood that although the first vital sign information and the second vital sign information are obtained from different measurement units, they can all include one or more values of respiration, heartbeat, pulse, blood pressure, and body temperature. Although the first movement information and the second movement information are also obtained by different measurement units, they can both include one or more of the limb movement of the measured object, the limb movement of the nurse, or the movement of the obstacle. So, after receiving the first measurement signal from the first measurement unit 11, the processor 13 can extract the first vital sign information and the first movement information from it. Moreover, after receiving the second measurement signal from the second measurement unit 12, the processor 13 can extract the second vital sign information and the second movement information from it. Afterwards, the processor 13 conducts comprehensive information analysis and calculation on the first vital sign information, the first movement information, the second vital sign information, and the second movement information. For example, the processor 13 corrects the first vital sign information and the second vital sign information, by using the first movement information and the second movement information, so as to obtain the physiological parameter of the measured object.

In a specific embodiment, refer to FIGS. 1, 6, and 7, an information processing method, that enables the processor 13 to correct at least one of the first vital sign information and the second vital sign information, by using at least one of the first movement information and the second movement information, so as to obtain the physiological parameter of the measured object, is provided. Specifically, the processor 13 analyzes the first vital sign information and the second vital sign information to obtain second vital sign data through an information quantification comparison. Moreover, the processor 13 processes the first movement information and the second movement information to obtain final movement information, and then corrects the second vital sign data by using the final movement information, so as to obtain the physiological parameter of the measured object.

Among them, the process of obtaining the second vital sign data through information quantification comparison can be divided into two optional situations. In optional situation (1), the processor 13 calculates an information confidence level which corresponds to the first vital sign information and the second vital sign information, respectively, such as calculating an information confidence level (or information quality) of the first vital sign information according to an amplitude, frequency, and time-varying characteristic of a radar waveform signal measured by a radar device, and calculating an information confidence level (or information quality) of second vital sign information according to an amplitude, frequency, and time-varying characteristic of an optical image signal measured by an optical camera, and takes the vital sign information which corresponds to a higher information confidence level in the first vital sign information and the second vital sign information as the second vital sign data. In optional situation (2), the processor 13 performs an information weighted fusion calculation on the first vital sign information and the second vital sign information, such as multiplying the first vital sign information with its corresponding weighting coefficient, multiplying the second vital sign information with its corresponding weighting coefficient and adding the products, to obtain the second vital sign data.

Among them, the process of obtaining the final movement information can be divided into two optional situations. In optional situation (1), the processor 13 calculates an information confidence level, which corresponds to the first movement information and the second movement information, respectively, such as calculating an information confidence level of the first movement information according to an amplitude, frequency, and time-varying characteristic of a radar waveform signal measured by a radar device, and calculating an information confidence level of the second movement information according to an amplitude, frequency, and time-varying characteristic of an optical image signal measured by an optical camera, and takes the movement information which corresponds to a higher information confidence level in the first movement information and the second movement information as the final movement information. In optional situation (2), the processor 13 performs an information weighted fusion calculation on the first movement information and the second movement information, such as multiplying the first movement information with its corresponding weighting coefficient, multiplying the second movement information with its corresponding weighting coefficient and adding the products, to obtain the final movement information.

Among them, the process of obtaining the physiological parameter through information correction can be divided into two optional situations. In optional situation (1), the processor 13 adjusts a numerical value of the second vital sign data by using the final movement information, such as subtracting the final movement information (or a product of the final movement information and an influence factor) from the second vital sign data and taking the obtained quantitative information as the physiological parameter of the measured object. In optional situation (2), the processor 13 utilizes the final movement information to maintain the second vital sign data at a fixed value for a preset time period, and if the final movement information is greater than a preset movement threshold, it indicates that the interference effect is severe, and the second vital sign data can be maintained at the previous value for a following preset time period (such as within 10 seconds) to avoid the occurrence of obvious inaccuracy of the vital sign information caused by the interference effect.

In a specific embodiment, please refer to FIGS. 1, 6, and 8, another information processing method, that enables the processor 13 to correct at least one of the first vital sign information and the second vital sign information by using at least one of the first movement information and the second movement information, is provided. Specifically, the processor 13 analyzes the first vital sign information and the second vital sign information to obtain the second vital sign data through an information quantification comparison. Then, the processor 13 corrects the second vital sign data, by using one of the first movement information and the second movement information, to obtain third vital sign data. Afterwards, the processor 13 corrects the third vital sign data, by using the other one of the first movement information and the second movement information to obtain fourth vital sign data. Finally, the processor 13 obtains the physiological parameter of the measured object according to the third vital sign data or the fourth vital sign data. As shown in FIG.5, the second vital sign data is corrected, by using the first movement information, to obtain the third vital sign data; and the third vital sign data is corrected, by using the second movement information, to obtain the fourth vital sign data; and the fourth vital sign data is taken as the physiological parameter of the measured object.

Among them, the process of obtaining the second vital sign data through information quantification comparison can be divided into two optional situations. In optional situation (1), the processor 13 calculates an information confidence level which corresponds to the first vital sign information and the second vital sign information, respectively, such as calculating an information confidence level (or information quality) according to an amplitude, frequency, and time-varying characteristic of a measured signal, and takes the vital sign information which corresponds to a higher information confidence level in the first vital sign information and the second vital sign information as the second vital sign data. In optional situation (2), the processor 13 performs an information weighted fusion calculation on the first vital sign information and the second vital sign information, such as multiplying the first vital sign information with its corresponding weighting coefficient, multiplying the second vital sign information with its corresponding weighting coefficient and adding the products, to obtain the second vital sign data.

The process of obtaining the third vital sign data through information correction can be divided into two optional situations. In optional situation (1), the processor 13 adjusts a numerical value of the second vital sign data according to the first movement information (or the second movement information) to obtain the third vital sign data. In optional situation (2), the processor 13 maintains the second vital sign data at a fixed value for a predetermined time period according to the first movement information (or the second movement information) to obtain the third vital sign data.

The process of obtaining the fourth vital sign data through information correction can be divided into two optional situations. In optional situation (1), the processor 13 adjusts a numerical value of the third vital sign data according to the second movement information (or the first movement information) to obtain the fourth vital sign data. In optional situation (2), the processor 13 maintains the third vital sign data at a fixed value for a predetermined time period according to the second movement information (or the first movement information) to obtain the fourth vital sign data.

In a specific embodiment, referring to FIGS. 1, 6, and 9, another information processing method is provided, which enables the processor 13 to correct at least one of the first vital sign information and the second vital sign information by using at least one of the first movement information and the second movement information. Specifically, the processor 13 corrects the first vital sign information by using the first movement information to obtain fifth vital sign data. Moreover, the processor 13 corrects the second vital sign information by using the second movement information to obtain sixth vital sign data. Then, processor 13 analyzes the fifth vital sign data and the sixth vital sign data to obtain the physiological parameter of the measured object through an numerical quantification comparison.

The process of obtaining the fifth vital sign data through information correction can be divided into two optional situations. In optional situation (1), the processor 13 adjusts a numerical value of the first vital sign information according to the first movement information to obtain the fifth vital sign data. In optional situation (2), the processor 13 maintains the first vital sign information at a fixed value for a predetermined time period according to the first movement information to obtain the fifth vital sign data.

The process of obtaining the sixth vital sign data through information correction can be divided into two optional situations. In optional situation (1), the processor 13 adjusts a numerical value of the second vital sign information according to the second movement information to obtain the sixth vital sign data. In optional situation (2), the processor 13 maintains the second vital sign information at a predetermined time period according to the second movement information to obtain the sixth vital sign data.

Among them, the process of obtaining the physiological parameter of the measured object through a numerical quantification comparison can be divided into three optional situations. In optional situation (1), the processor 13 normalizes the first movement information and the second movement information to obtain a corresponding interference amplitude, wherein the normalization processing can be considered as converting the first movement information and the second movement information to the same amplitude level to ensure an unity of amplitude unit, such that the vital sign data with a smaller interference amplitude, in the fifth vital sign data and the sixth vital sign data, is taken as the physiological parameter of the measured object, wherein the fifth vital sign data is obtained by participating the first movement information into its correction, and the sixth vital sign data is obtained by participating the second movement information into its correction. In optional situation (2), the processor 13 calculates an information confidence level which corresponds to the first vital sign information and the second vital sign information, such as calculating an information confidence level according to an amplitude, frequency, and time-varying characteristic of a measured signal, and takes the vital sign data, which corresponds to a higher information confidence level in the fifth vital sign data and the sixth vital sign data, as the physiological parameter of the measured object, wherein the fifth vital sign data is obtained by correcting the first vital sign information, and the sixth vital sign data is obtained by correcting the second vital sign information. In optional situation (3), the processor 13 performs an information weighted fusion calculation on the fifth vital sign data and the sixth vital sign data, such as multiplying the fifth vital sign data with its corresponding weighting coefficient, multiplying the sixth vital sign data with its corresponding weighting coefficient and adding the products, so as to obtain the physiological parameter of the measured object.

In a specific embodiment, referring to FIGS. 1, 6, and 10, a different information processing method is further provided, which enables the processor 13 to correct at least one of the first vital sign information and the second vital sign information by using at least one of the first movement information and the second movement information. Specifically, the processor 13 corrects one of the first vital sign information and the second vital sign information to obtain seventh vital sign data by using one of the first movement information and the second movement information, and analyzes uncorrected vital sign information in the first vital sign information and the second vital sign information with the seventh vital sign data, so as to obtain the physiological parameter of the measured object through a numerical quantification comparison.

The process of obtaining the seventh vital sign data through information correction can be divided into two optional situations. In optional situation (1), the processor 13 adjusts a numerical value of the first vital sign information according to the first movement information (or the second movement information) to obtain the seventh vital sign data. In optional situation (2), the processor 13 maintains the first vital sign information at a predetermined time period according to the first movement information (or the second movement information) to obtain the seventh vital sign data.

Among them, the process of obtaining the physiological parameter of the measured object through a numerical quantification comparison can be divided into two optional situations. In optional situation (1), the processor 13 calculates an information confidence level which corresponds to the seventh vital sign data and the second vital sign information, respectively, such as calculating an information confidence level according to an amplitude, frequency, and time-varying characteristic of a measured signal, and takes the vital sign data (or the vital sign information) which corresponds to a higher information confidence level in the seventh vital sign data which is corrected by the first vital sign information and the second vital sign information as the physiological parameter of the measured object. In optional situation (2), the processor 13 performs an information weighted fusion calculation on the seventh vital sign data and the second vital sign information, such as multiplying the seventh vital sign data with its corresponding weighting coefficient, multiplying the second vital sign information with its corresponding weighting coefficient and adding the products, so as to obtain the physiological parameter of the measured object.

It should be noted that for the situations in FIGS. 4 and 7, the processor 13 needs to process according to the first movement information and the second movement information to obtain the final movement information. According to the specific embodiments mentioned above, it can be divided into two implementation scenarios. In scenario 1, the processor 13 calculates an information confidence level which corresponds to the first movement information and the second movement information, respectively, such as calculating the information confidence level according to an amplitude, frequency, and time-varying characteristic of a measured signal, and takes the movement information which corresponds to a higher information confidence level in the first movement information and the second movement information as the final movement information. In Scenario 2, the processor 13 performs an information weighted fusion calculation on the first movement information and the second movement information, such as multiplying the first movement information with its corresponding weighting coefficient, multiplying the second movement information with its corresponding weighting coefficient and adding the products, to obtain the final movement information.

It should be noted that the correction made to the first vital sign information and/or the second vital sign information mentioned earlier include adjusting a magnitude of a value or maintaining a value unchanged for a predetermined time.

For example, for the situations in FIGS. 4, 5, 9, and 10, the processor 13 needs to correct vital sign information (such as the first vital sign information). The process of performing the correction can be summarized as follows. Firstly, the processor 13 acquires the vital sign information and movement information participating in the correction. Then, processor 13 adjusts the numerical value of the vital sign information participating in the correction according to the movement information participating in the correction, or the processor 13 maintains the numerical value of the vital sign information participating in the correction at a fixed value for a predetermined time period according to the movement information participating in the correction. Among them, in order to adjust the numerical value of the vital sign information participating in the correction, the movement information participating in the correction (or the product of the movement information participating in the correction and its influence factor) can be subtracted from the vital sign information participating in the correction, and the quantified information obtained after calculation is the adjusted value. Among them, in order to maintain the numerical value of the vital sign information participating in the correction unchanged within the preset time period, when the movement information participating in the correction is greater than a preset movement threshold, the vital sign information participating in the correction is maintained at the previous value in the following preset time period (such as within 10 seconds), so as to avoid the occurrence of obvious inaccuracy of the vital sign information caused by interference.

For example, for the situations in FIGS. 5, 7, and 8, the processor 13 needs to correct the vital sign data. Therefore, the process of performing the correction can be summarized as follows. Firstly, the processor 13 acquires the vital sign data and movement information participating in the correction. Then, processor 13 adjusts the numerical value of the vital sign data participating in the correction according to the movement information participating in the correction, or the processor 13 maintains the vital sign data participating in the correction at a fixed value for a predetermined time period according to the movement information participating in the correction. Among them, in order to adjust the numerical value of the vital sign data participating in the correction, the movement information participating in the correction (or the product of the movement information participating in the correction and its influence factor) can be subtracted from the vital sign information participating in the correction, and the quantified information obtained after calculation is the adjusted value. Among them, in order to maintain the numerical value of the vital sign information participating in the correction unchanged within the preset time period, when the movement information participating in the correction is greater than the preset movement threshold, the vital sign information participating in the correction is maintained at the previous value in the following preset time period (such as within 10 seconds), so as to avoid the occurrence of obvious inaccuracy of the vital sign information caused by interference.

It can be understood that the physiological parameter monitoring system above uses two measurement units for measuring vital sign information and movement information. Adding the second measurement unit can overcome the problem of poor anti-interference performance when using the radar device alone for non-contact vital sign measurement. Since the first measurement unit can measure the first vital sign information and/or the first movement information, and the second measurement unit can measure the second vital sign information and/or the second movement information, by selecting, fusing or correcting the information measured by different measurement units, these information can participate the processing of vital sign information, thus achieving information correction in various ways, and enhancing the reliability of the system, so as to obtain more accurate measurements of vital signs. On the basis of the physiological parameter monitoring system disclosed in FIGS. 1 to 10, this disclosure also discloses a physiological parameter monitoring method, which is mainly applied at the processor 13 in FIG. 1.

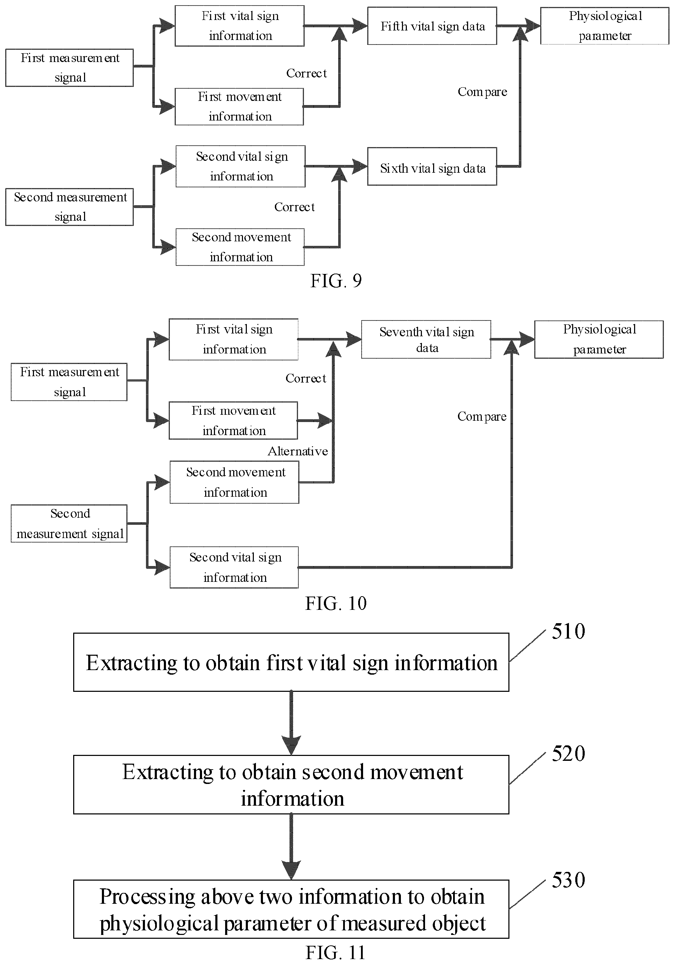

In one embodiment, please refer to FIG. 11. The disclosed physiological parameter monitoring method includes steps 510-530, and is explained separately below.

In step 510, a measurement signal of one measurement unit is extracted, such that first vital sign information of a measured object is extracted from the measurement signal of one measurement unit. For example, in FIG. 1, since the first measurement unit 11 can be located around the measured object (such as a patient, newborn, etc.), non-contact measurement is performed on the measured object to obtain the first measurement signal. Therefore, the processor 13 can receive the first measurement signal from the first measurement unit 11 and extract the first vital sign information of the measured object from the first measurement signal.

It should be noted that one measurement unit (such as the first measurement unit 11) herein can use a radar device. Due to the more mature and effective radar detection technology, it can be used as the main device for vital sign measurement. Of course, the first vital sign information extracted from the first measurement signal, can include one or more of respiration, heartbeat, pulse, blood pressure, and body temperature, all of which can reflect the vital sign state of the measured object. For example, the vital sign information taking respiration as an example includes but is not limited to a respiratory signal (respiratory wave), respiratory rate, respiratory amplitude, inhalation or exhalation status, whether apnea occurs, whether suffocation occurs, and number of occurrence times of apnea or suffocation per unit time.

In step 520, a measurement signal of another measurement unit is extracted, such that second movement information, which is caused by an movement that affects vital sign of the measured object, can be extracted from the measurement signal of the another measurement unit. For example, in FIG. 1, since the second measurement unit 12 is located around the measured object to perform the non-contact measurement on the measured object to obtain the second measurement signal, the processor 13 can receive the second measurement signal from the second measurement unit 12 and extract the second movement information which is caused by the movement that affect the vital sign of the measured object.

It should be noted that the another measurement unit (such as the second measurement unit 12) can use one or more of: a radar device, an optical camera, an infrared sensor, an infrared thermal imager, a depth measurement sensor, a sound sensor, a pressure sensor, and an acceleration sensor. Due to the adverse effects of limb movements of the measured object, wearing movements of the measured object, and rolling movements on the measured object of nurse on the vital sign measurement, the second movement information which is extracted from the second measurement signal includes one or more of a limb movement of the measured object, a limb movement of a nurse, and a movement of an obstacle. For example, taking the limb movement of the measured object as an example, the movement information includes but is not limited to whether interference movement occurs, magnitude of interference, and a duration of interference, etc.

In step 530, at least the first vital sign information is corrected according to the second movement information, so as to obtain the physiological parameter of the measured object. Due to the fact that the first vital sign information and the second movement information are the results of simultaneous measurement of the measured object by using different measurement units, the interference situations can also have an impact on the value of the first vital sign information. Therefore, it is necessary to use the second movement information to correct the first vital sign information and calculate a more accurate vital sign measurement result. It can be understood that the physiological parameter of the measured object is a quantified numerical result or fluctuation curve, which corresponds to the content of the first vital sign information. The physiological parameter of the measured object can specifically include one or more values of: respiration, heartbeat, pulse, blood pressure, and body temperature, making it easy for users to directly understand the vital sign state of the measured object.

For example, in FIG. 1, the processor 13 can only correct the first vital sign information in the first measurement signal according to the second movement information in the second measurement signal. In an optional situation 1, the processor 13 adjusts the numerical value of the first vital sign information by using the second movement information. For example, the quantified information, which is obtained by subtracting the second movement information (or the product of the second movement information and its influence factor) from the first vital sign information, can be the physiological parameter of the measured object. In an optional situation 2, the processor 13 utilizes the second movement information to maintain the first vital sign information at a fixed value for a preset time period. If the second movement information is greater than the preset movement threshold, it indicates that the interference effect is severe. The first vital sign information can be maintained at the previous value for the following preset time period (such as within 10 seconds) to avoid the occurrence of obvious inaccuracy of the vital sign information caused by the interference effect.

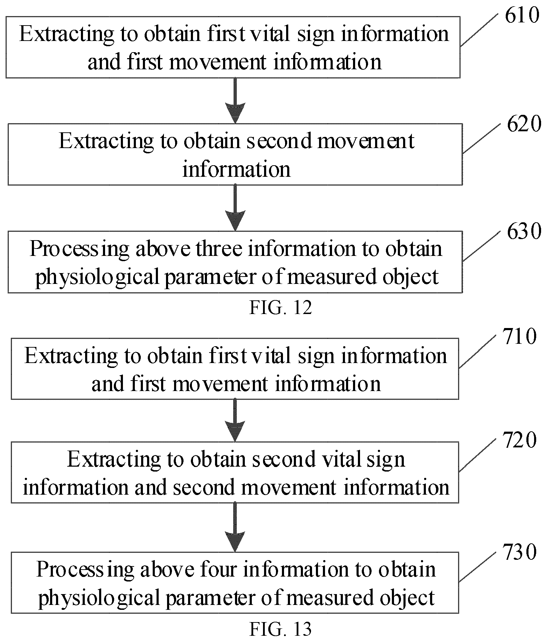

In one embodiment, please refer to FIG. 12. The disclosed physiological parameter monitoring method includes steps 610-640, and is explained separately below.

Compared to step 510, there are some differences in step 610. Not only the first vital sign information is extracted from the measurement signal of one measurement unit (such as the first measurement signal of the first measurement unit 11 in FIG. 1), but also the first movement information which is caused by the movement that affects the measurement of the vital sign of the measured object is extracted from the measurement information. It can be understood that the first vital sign information includes one or more of respiration, heartbeat, pulse, blood pressure, and body temperature, all of which can reflect the vital sign state of the measured object. The first movement information includes one or more of: a limb movement of the measured object, a limb movement of a nurse, and a movement of an obstacle.

Step 620, similar to step 520, in which the second movement information, which is caused by the movement that affects the vital sign of the measured object, can be extracted from the measurement signal of the other measurement unit (such as the second measurement signal of the second measurement unit 12 in FIG. 1). It can be understood that the second movement information includes one or more of: a limb movement of the measured object, a limb movement of a nurse, and a movement of an obstacle.

In step 630, the first vital sign information is corrected according to the first movement information and the second movement information, so as to obtain the physiological parameter of the measured object. For example, in FIG. 1 and FIG.3, the processor 13 conducts comprehensive information analysis and calculation according to the first vital sign information, the first movement information, and the second movement information. Specifically, the first vital sign information is corrected, by using the first movement information and the second movement information, so as to obtain the physiological parameter of the measured object.

The information processing method in step 630 can be explained in detail by using FIGS. 4 and 5, respectively.

For example, FIG. 4 provides an information processing method that enables the processor 13 to correct the first vital sign information according to the first movement information and the second movement information. Specifically, the final movement information is obtained by processing the first movement information and the second movement information; and then, the final movement information is used to correct the first vital sign information, so as to obtain the physiological parameter of the measured object.

Among them, the process of obtaining the final movement information can be divided into two optional situations. In optional situation (1), an information confidence level which corresponds to the first movement information and the second movement information are calculated respectively, such as calculating an information confidence level of the first movement information according to an amplitude, frequency, and time-varying characteristic of a radar waveform signal measured by a radar device, and calculating an information confidence level of the second movement information according to an amplitude, frequency, and time-varying characteristic of an optical image signal measured by an optical camera, and then the movement information which corresponds to a higher information confidence level in the first movement information and the second movement information, is taken as the final movement information. In optional situation (2) , an information weighted fusion calculation is performed on the first movement information and the second movement information, such as multiplying the first movement information with its corresponding weighting coefficient, multiplying the second movement information with its corresponding weighting coefficient and adding the products, to obtain the final movement information.

Among them, the process of obtaining the physiological parameter through the information correction can be divided into two optional situations. In optional situation (1), a numerical value of the first vital sign information is adjusted by using the final movement information, so as to obtain the physiological parameter of the measured object, such as subtracting the final movement information (or a product of the final movement information and an influence factor) from the first vital sign information and taking the obtained quantitative information as the physiological parameter of the measured object. In optional situation (2), the final movement information is utilized to maintain the first vital sign information at a fixed value for a preset time period, and if the final movement information is greater than a preset movement threshold, it indicates that the interference effect is severe, and the first vital sign information can be maintained at the previous value for a following preset time period (such as within 10 seconds) to avoid the occurrence of obvious inaccuracy of the vital sign information caused by the interference effect.

For example, FIG. 5 provides another information processing method that enables the processor 13 to correct the first vital sign information by using the first movement information and the second movement information. Specifically, the first vital sign information is corrected, by using one of the first movement information and the second movement information, to obtain first vital sign data; and the first vital sign data is corrected, by using the other one of the first movement information and the second movement information, so as to obtain the physiological parameter of the measured object.

The process of obtaining the first vital sign data through information correction can be divided into two optional situations. In optional situation (1), a numerical value of the first vital sign information is adjusted according to the first movement information (or the second movement information) to obtain the first vital sign data; (2) a numerical value of the first vital sign information is maintained at a fixed value for a predetermined time period according to the first movement information (or the second movement information) to obtain the first vital sign data.

The process of obtaining the physiological parameter through information correction can be divided into two optional situations. In optional situation (1), a numerical value of the first vital sign data is adjusted according to the second movement information (or the first movement information), so as to obtain the physiological parameter of the measured object. In optional situation (2), the first vital sign data is maintained at a fixed value for a predetermined time period according to the second movement information (or the first movement information), so as to obtain the physiological parameter of the measured object.

In one embodiment, please refer to FIG. 13. The disclosed physiological parameter monitoring method includes steps 710-730, and is explained separately below.

Compared to step 510, there are some differences in step 710. Not only the first vital sign information is extracted from the measurement signal of one measurement unit (such as the first measurement signal of the first measurement unit 11 in FIG. 1), but also first movement information which is caused by the movement that affects the measurement of the vital sign of the measured object is extracted from the measurement information. It can be understood that the first vital sign information includes one or more of respiration, heartbeat, pulse, blood pressure, and body temperature, all of which can reflect the vital sign state of the measured object. The first movement information includes one or more of: a limb movement of the measured object, a limb movement of a nurse, and a movement of an obstacle.

Compared to step 520, there are some differences in step 720. Not only the second movement information caused by the movement that affects the vital sign of the measured object is extracted from the measurement signal of the another measurement unit (such as the second measurement signal of the second measurement unit 12 in FIG. 1), but also second vital sign data is extracted from the measurement signal. It can be understood that the second movement information includes one or more of: a limb movement of the measured object, a limb movement of a nurse, and a movement of an obstacle, and the second vital sign information includes one or more of respiration, heartbeat, pulse, blood pressure, and body temperature.

In step 730, at least one of the first vital sign information and the second vital sign information are corrected according to at least one of the first movement information and the second movement information, so as to obtain the physiological parameter of the measured object. For example, in FIG. 1 and FIG.6, the processor 13 conducts comprehensive information analysis and calculation according to the first vital sign information, the first movement information, the second vital sign information, and the second movement information. Specifically, the first vital sign information and the second vital sign information are corrected, by using the first movement information and the second movement information, so as to obtain the physiological parameter of the measured object.

The information processing method in step 730 can be explained in detail by using FIGS. 7, 8, 9, and 10, respectively.

For example, FIG. 7 provides an information processing method that enables the processor 13 to correct at least one of the first vital sign information and the second vital sign information according to at least one of the first movement information and the second movement information, so as to obtain the physiological parameter of the measured object. Specifically, the first vital sign information and the second vital sign information are analyzed to obtain second vital sign data through an information quantification comparison; the first movement information and the second movement information are processed to obtain final movement information; and the second vital sign data is corrected by the final movement information, so as to obtain the physiological parameter of the measured object.

Among them, the process of obtaining the second vital sign data through information quantification comparison can be divided into two optional situations. In optional situation (1), an information confidence level which corresponds to the first vital sign information and the second vital sign information, is calculated respectively, such as calculating an information confidence level (or information quality) of the first vital sign information according to an amplitude, frequency, and time-varying characteristic of a radar waveform signal measured by a radar device, and calculating an information confidence level (or information quality) of second vital sign information according to an amplitude, frequency, and time-varying characteristic of an optical image signal measured by an optical camera; and the vital sign information which corresponds to a higher information confidence level in the first vital sign information and the second vital sign information, is taken as the second vital sign data. In optional situation (2), an information weighted fusion calculation is performed on the first vital sign information and the second vital sign information, such as multiplying the first vital sign information with its corresponding weighting coefficient, multiplying the second vital sign information with its corresponding weighting coefficient and adding the products, to obtain the second vital sign data.

Among them, the process of obtaining the final movement information can be divided into two optional situations. In optional situation (1), an information confidence level, which corresponds to the first movement information and the second movement information, respectively, is calculated, such as calculating an information confidence level of the first movement information according to an amplitude, frequency, and time-varying characteristic of a radar waveform signal measured by a radar device, and calculating an information confidence level of the second movement information according to an amplitude, frequency, and time-varying characteristic of an optical image signal measured by an optical camera; and the movement information, which corresponds to a higher information confidence level in the first movement information and the second movement information, is taken as the final movement information. In optional situation (2), an information weighted fusion calculation is performed on the first movement information and the second movement information, such as multiplying the first movement information with its corresponding weighting coefficient, multiplying the second movement information with its corresponding weighting coefficient and adding the products, to obtain the final movement information.

The process of obtaining the physiological parameter through information correction can be divided into two optional situations. In optional situation (1), a numerical value of the second vital sign data is adjusted to obtain the physiological parameter of the measured object, by using the final movement information, such as taking quantitative information which is obtained by subtracting the final movement information (or the product of the final movement information and its influence factor) from the first vital sign data as the physiological parameter of the measured object. In optional situation (2), the second vital sign data is maintained at a fixed value for a predetermined time period by using the final movement information. For example, if the final movement information is greater than the preset movement threshold, it indicates that the interference effect is relatively serious. The second vital sign data can be maintained at the previous value for the following preset time period (such as within 10 seconds) to avoid the occurrence of obvious inaccurate sign information caused by the interference effect.

For example, FIG. 8 provides another information processing method that enables the processor 13 to correct at least one of the first vital sign information and the second vital sign information, by using at least one of the first movement information and the second movement information, so as to obtain the physiological parameter of the measured object. Specifically, the first vital sign information and the second vital sign information are analyzed to obtain second vital sign data through an information quantification comparison. Moreover, the second vital sign data is corrected to obtain third vital sign data by using one of the first movement information and the second movement information. Afterwards, the third vital sign data is corrected to obtain fourth vital sign data, by using the other one of the first movement information and the second movement information. Finally, the physiological parameter of the measured object is obtained according to the third vital sign data or the fourth vital sign data. As shown in FIG. 5, the second vital sign data is corrected, by using the first movement information, to obtain the third vital sign data; and the third vital sign data is corrected, by using the second movement information, to obtain the fourth vital sign data, and the fourth vital sign data is taken as the physiological parameter of the measured object.

Among them, the process of obtaining the second vital sign data through information quantification comparison can be divided into two optional situations. In optional situation (1), an information confidence level which corresponds to the first vital sign information and the second vital sign information, is calculated respectively, such as calculating an information confidence level (or information quality) of the first vital sign information according to an amplitude, frequency, and time-varying characteristic of a measured signal; and the vital sign information which corresponds to a higher information confidence level in the first vital sign information and the second vital sign information, is taken as the second vital sign data. In optional situation (2), an information weighted fusion calculation is performed on the first vital sign information and the second vital sign information, such as multiplying the first vital sign information with its corresponding weighting coefficient, multiplying the second vital sign information with its corresponding weighting coefficient and adding the products, to obtain the second vital sign data.

Among them, the process of obtaining the third vital sign data through information quantification comparison can be divided into two optional situations. In optional situation (1), a numerical value of the second vital sign data is adjusted, according to the first movement information (or the second movement information), to obtain the third vital sign data. In optional situation (2), the second vital sign data is maintained at a fixed value for a predetermined time period, according to the first movement information (or the second movement information), to obtain the third vital sign data.

Among them, the process of obtaining the fourth vital sign data through information quantification comparison can be divided into two optional situations. In optional situation (1), a numerical value of the third vital sign data is adjusted, according to the second movement information (or the first movement information), to obtain the fourth vital sign data. In optional situation (2), the third vital sign data is maintained at a fixed value for a predetermined time period, according to the second movement information (or the first movement information), to obtain the fourth vital sign data.

For example, FIG. 9 provides another information processing method that enables the processor 13 to correct at least one of the first vital sign information and the second vital sign information, by using at least one of the first movement information and the second movement information, so as to obtain the physiological parameter of the measured object. Specifically, the first vital sign information is corrected to obtain fifth vital sign data, by using the first movement information and the second vital sign information is corrected to obtain sixth vital sign data, by using the second movement information; and then the fifth vital sign data and the sixth vital sign data are analyzed, so as to obtain the physiological parameter of the measured object through an numerical quantification comparison.

Among them, the process of obtaining the fifth vital sign data through information correction can be divided into two optional situations. In optional situation (1), a numerical value of the first vital sign information is adjusted by using the first movement information to obtain the fifth vital sign data. In optional situation (2), the first vital sign information is maintained at a fixed value for a predetermined time period, according to the first movement information, to obtain the fifth vital sign data.

Among them, the process of obtaining the sixth vital sign data through information correction can be divided into two optional situations. In optional situation (1), a numerical value of the second vital sign information is adjusted by using the second movement information to obtain the sixth vital sign data. In optional situation (2), the second vital sign information is maintained at a fixed value for a predetermined time period, according to the second movement information, to obtain the sixth vital sign data.

Among them, the process of obtaining the physiological parameter of the measured object through a numerical quantification comparison can be divided into three optional situations. In optional situation (1), the first movement information and the second movement information are normalized to obtain a corresponding interference amplitude, wherein the normalization processing can be considered as converting the first movement information and the second movement information to the same amplitude level to ensure an unity of amplitude unit, such that the vital sign data with a smaller interference amplitude, in the fifth vital sign data and the sixth vital sign data, is taken as the physiological parameter of the measured object, wherein the fifth vital sign data is obtained by participating the first movement information into its correction, and the sixth vital sign data is obtained by participating the second movement information into its correction. In optional situation (2), an information confidence level which corresponds to the first vital sign information and the second vital sign information, is calculated respectively, such as calculating an information confidence level according to an amplitude, frequency, and time-varying characteristic of a measured signal, and the vital sign data, which corresponds to a higher information confidence level in the fifth vital sign data and the sixth vital sign data, is taken as the physiological parameter of the measured object, wherein the fifth vital sign data is obtained by correcting the first vital sign information, and the sixth vital sign data is obtained by correcting the second vital sign information. In optional situation (3), an information weighted fusion calculation is performed on the fifth vital sign data and the sixth vital sign data, such as multiplying the fifth vital sign data with its corresponding weighting coefficient, multiplying the sixth vital sign data with its corresponding weighting coefficient and adding the products, so as to obtain the physiological parameter of the measured object.

For example, FIG. 10 further provides an information processing method, that enables the processor 13 to correct at least one of the first vital sign information and the second vital sign information, by using at least one of the first movement information and the second movement information, so as to obtain the physiological parameter of the measured object, is provided. Specifically, one of the first vital sign information and the second vital sign information is corrected, by using one of the first movement information and the second movement information, to obtain seventh vital sign data; and then the uncorrected vital sign information in the first vital sign information and the second vital sign information is analyzed with the seventh vital sign data, so as to obtain the physiological parameter of the measured object through a numerical quantification comparison.

Among them, the process of obtaining the seventh vital sign data through information correction can be divided into two optional situations. In optional situation (1), a numerical value of the first vital sign information is adjusted by using the first movement information (or the second movement information) to obtain the seventh vital sign data. In optional situation (2), the first vital sign information is maintained at a fixed value for a predetermined time period, according to the first movement information (or the second movement information), to obtain the seventh vital sign data.

Among them, the process of obtaining the physiological parameter of the measured object through a numerical quantification comparison can be divided into three optional situations. In optional situation (1), an information confidence level which corresponds to the seventh vital sign data and the second vital sign information, is calculated respectively, such as calculating an information confidence level according to an amplitude, frequency, and time-varying characteristic of a measured signal, and the vital sign data (or the vital sign information), which corresponds to a higher information confidence level in the seventh vital sign data and the second vital sign information, is taken as the physiological parameter of the measured object. In optional situation (2), an information weighted fusion calculation is performed on the seventh vital sign data and the second vital sign information, such as multiplying the seventh vital sign data with its corresponding weighting coefficient, multiplying the second vital sign information with its corresponding weighting coefficient and adding the products, so as to obtain the physiological parameter of the measured object. Based on the information processing method disclosed in FIGS. 11-13, an information processing device is further disclosed.

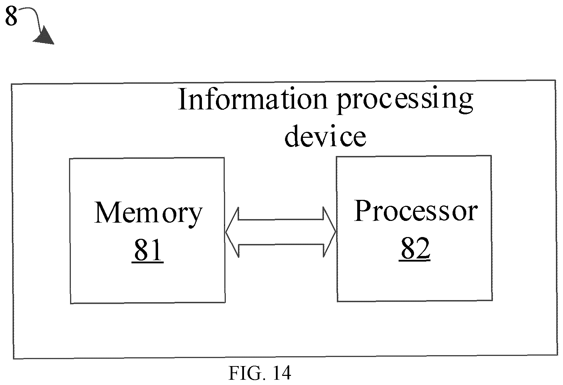

Please refer to FIG. 14. The disclosed information processing device 8 may include a memory 81 and a processor 82. It can be understood that the memory 81 and processor 82 here are main components of the information processing device 8. Of course, the information processing device 8 can also include some measurement components connected to the processor 82, such as the first measurement unit 11 and the second measurement unit 12 in FIG. 1, which are not explained in detail here.

Among them, the memory 81 can be used as a computer-readable storage medium for storing programs, which can be program code corresponding to the physiological parameter monitoring method mentioned above.

Among them, processor 82 is connected to the memory 81 for executing programs stored in the memory 81 to implement the monitoring method disclosed in the above embodiment, as detailed in steps 510-530 in FIG. 11, steps 610-630 in FIG. 12, and steps 710-730 in FIG. 13. It should be noted that the functions implemented by the processor 82 can be referred to the processor 13 in FIG. 1, and are not explained in detail here.

One skilled in the art can understand that all or part of the functions of various methods in the above embodiments can be achieved through hardware or computer programs. When all or part of the functions in the above embodiments are implemented through a computer program, the program can be stored in a computer-readable storage medium, which can include read-only memory, random access memory, magnetic disk, optical disk, hard disk, etc. The program is executed by a computer to achieve the above functions. For example, the program can be stored in a memory of a device, and executed by the processor to achieve all or part of the above functions. In addition, when all or part of the functions in the above embodiments are implemented through a computer program, the program can also be stored on a storage media such as a server, another computer, a disk, CD, flash drive, or mobile hard drive, and be downloaded or copied to the memory of a local device or to update system version of the local device. When the program in the memory is executed through the processor, all or part of the functions in the above implementation can be achieved.

The above specific examples are to explain this disclosure, and they are only for the purpose of assisting in understanding the technical solution of this disclosure, and is not intended to limit this disclosure. For technical personnel in the present technical field, based on the ideas of this disclosure, several simple deductions, deformations, or replacements can also be made.

## Claims

1. A physiological parameter monitoring system, **characterized in that**, comprising:
a first measurement unit, which is configured to implement non-contact measurement on a measured object to acquire a first measurement signal, wherein the first measurement signal comprises at least first vital sign information of the measured object;
a second measurement unit, which is configured to implement non-contact measurement on the measured object to acquire a second measurement signal, wherein the second measurement signal at least comprises second movement information which is caused by a movement that affects a vital sign of the measured object; and
a processor which is configured to acquire the first measurement signal of the first measurement unit and extract the first vital sign information from the first measurement signal, to acquire the second measurement signal of the second measurement unit and extract the second movement information from the second measurement signal, and to at least correct the first vital sign information according to the second movement information, so as to obtain a physiological parameter of the measured object.

2. The physiological parameter monitoring system according to claim 1, **characterized in that**, the first measurement signal further comprises first movement information which is caused by the movement that affects the vital sign of the measured object, and the processor is further configured to extract the first movement information from the first measurement signal;
wherein in order to correct the first vital sign information according to the second movement information, so as to obtain a physiological parameter of the measured object, the processor is further configured to correct the first vital sign information, at least according to the first movement information and the second movement information, so as to obtain the physiological parameter of the measured object;
preferably, the first vital sign information comprises one or more of respiration, heartbeat, pulse, blood pressure, and body temperature; wherein each of the first movement information and the second movement information comprises one or more of a limb movement of the measured object, a limb movement of a nurse, and a movement of an obstacle.

3. The physiological parameter monitoring system according to claim 2, **characterized in that**, in order to correct the first vital sign information, at least according to the first movement information and the second movement information, so as to obtain the physiological parameter of the measured object, the processor is further configured to:
process the first movement information and the second movement information to obtain final movement information; and
correct the first vital sign information by using the final movement information, so as to obtain the physiological parameter of the measured object;
preferably, in order to process the first movement information and the second movement information to obtain final movement information, the processor is further configured to perform an information weighted fusion calculation on the first movement information and the second movement information to obtain the final movement information.

4. The physiological parameter monitoring system according to claim 2, **characterized in that**, in order to correct the first vital sign information, at least according to the first movement information and the second movement information, so as to obtain the physiological parameter of the measured object, the processor is further configured to:
correct the first vital sign information, by using one of the first movement information and the second movement information, to obtain first vital sign data; and
correct the first vital sign data, by using the other one of the first movement information and the second movement information, so as to obtain the physiological parameter of the measured object.

5. The physiological parameter monitoring system according to any one of claims 1-4, **characterized in that**, the correction comprises correcting a magnitude of a value or maintaining a value unchanged for a predetermined time.

6. A physiological parameter monitoring system, **characterized in that**, comprising:
a first measurement unit, which is configured to implement non-contact measurement on a measured object to acquire a first measurement signal, wherein the first measurement signal comprises first vital sign information of the measured object and first movement information which is caused by a movement that affects a vital sign of the measured object;
a second measurement unit, which is configured to implement non-contact measurement on the measured object to acquire a second measurement signal, wherein the second measurement signal comprises second vital sign information of the measured object and second movement information which is caused by the movement that affects the vital sign of the measured object; and
a processor, which is configured to acquire the first measurement signal of the first measurement unit and extract the first vital sign information and the first movement information, to acquire the second measurement signal of the second measurement unit and extract the second vital sign information and the second movement information from the second measurement signal;
wherein the processor is further configured to correct at least one of the first vital sign information and the second vital sign information, according to at least one of the first movement information and the second movement information, so as to obtain a physiological parameter of the measured object.

7. The physiological parameter monitoring system according to claim 6, **characterized in that**, in order to correct at least one of the first vital sign information and the second vital sign information, according to at least one of the first movement information and the second movement information, so as to obtain a physiological parameter of the measured object, the processor is further configured to:
analyze the first vital sign information and the second vital sign information to obtain second vital sign data through an information quantification comparison;
process the first movement information and the second movement information to obtain final movement information; and
correct the second vital sign data by using the final movement information to obtain the physiological parameter of the measured object;
preferably, in order to process the first movement information and the second movement information to obtain final movement information, the processor is further configured to: calculate a respective information confidence level which corresponds to the first movement information and the second movement information, and take the movement information, which corresponds to a higher information confidence level in the first movement information and the second movement information, as the final movement information; or perform an information weighted fusion calculation on the first movement information and the second movement information to obtain the final movement information; or
preferably, in order to analyze the first vital sign information and the second vital sign information to obtain second vital sign data through an information quantification comparison, the processor is further configured to: calculate a respective information confidence level which corresponds to the first vital sign information and the second vital sign information, and take the vital sign information, which corresponds to a higher information confidence level in the first vital sign information and the second vital sign information, as the second vital sign data; or perform an information weighted fusion calculation on the first vital sign information and the second vital sign information to obtain the second vital sign data.

8. The physiological parameter monitoring system according to claim 6, **characterized in that**, in order to correct at least one of the first vital sign information and the second vital sign information, according to at least one of the first movement information and the second movement information, so as to obtain a physiological parameter of the measured object, the processor is further configured to:
analyze the first vital sign information and the second vital sign information to obtain second vital sign data through an information quantification comparison;
correct the second vital sign data, by using one of the first movement information and the second movement information, to obtain third vital sign data;
correct the third vital sign data, by using the other one of the first movement information and the second movement information, to obtain fourth vital sign data; and
obtain the physiological parameter of the measured object according to the third vital sign data or the fourth vital sign data;
preferably, in order to analyze the first vital sign information and the second vital sign information to obtain second vital sign data through an information quantification comparison, the processor is further configured to: calculate a respective information confidence level which corresponds to the first vital sign information and the second vital sign information, and take the vital sign information, which corresponds to a higher information confidence level in the first vital sign information and the second vital sign information, as the second vital sign data; or perform an information weighted fusion calculation on the first vital sign information and the second vital sign information to obtain the second vital sign data.

9. The physiological parameter monitoring system according to claim 6, **characterized in that**, in order to correct at least one of the first vital sign information and the second vital sign information, according to at least one of the first movement information and the second movement information, so as to obtain a physiological parameter of the measured object, the processor is further configured to:
correct the first vital sign information by using the first movement information to obtain fifth vital sign data;
correct the second vital sign information by using the second movement information to obtain sixth vital sign data; and
analyze the fifth vital sign data and the sixth vital sign data, so as to obtain the physiological parameter of the measured object through a numerical quantification comparison;
preferably, in order to analyze the fifth vital sign data and the sixth vital sign data, so as to obtain the physiological parameter of the measured object through a numerical quantification comparison, the processor is further configured to: normalize the first movement information and the second movement information respectively to obtain a corresponding interference amplitude, and take the vital sign data with a smaller interference amplitude in the fifth vital sign data and the sixth vital sign data as the physiological parameter of the measured object; or calculate a respective information confidence level which corresponds to the first vital sign information and the second vital sign information, and take the vital sign data, which corresponds to a higher information confidence level in the fifth vital sign data and the sixth vital sign data, as the physiological parameter of the measured object; or perform an information weighted fusion calculation on the fifth vital sign data and the sixth vital sign data, so as to obtain the physiological parameter of the measured object.

10. The physiological parameter monitoring system according to claim 6, **characterized in that**, in order to correct at least one of the first vital sign information and the second vital sign information, according to at least one of the first movement information and the second movement information, so as to obtain a physiological parameter of the measured object, the processor is further configured to:
correct one of the first vital sign information and the second vital sign information, by using one of the first movement information and the second movement information, to obtain seventh vital sign data; and
analyze uncorrected vital sign information in the first vital sign information and the second vital sign information with the seventh vital sign data, so as to obtain the physiological parameter of the measured object through a numerical quantification comparison.

11. The physiological parameter monitoring system according to claim 6, **characterized in that**, the correction comprises correcting a magnitude of a value or maintaining a value unchanged for a predetermined time; or
each of the first vital sign information and the second vital sign information comprises one or more of respiration, heartbeat, pulse, blood pressure, and body temperature; each of the first movement information and the second movement information comprises one or more of a limb movement of the measured object, a limb movement of a nurse, and a movement of an obstacle; or
the first measurement unit comprises a radar device; the second measurement unit comprises one or more of: a radar device, an optical camera, an infrared sensor, an infrared thermal imager, a depth measurement sensor, a sound sensor, a pressure sensor, and an acceleration sensor.

12. A physiological parameter monitoring method, **characterized in that**, comprising:
extracting first vital sign information of a measured object from a measurement signal of one measurement unit;
extracting second movement information, which is caused by a movement that affects a vital sign of the measured object, from a measurement signal of another measurement unit; and
at least correcting the first vital sign information according to the second movement information, so as to obtain a physiological parameter of the measured object.

13. The physiological parameter monitoring method according to claim 12, **characterized in that**, the physiological parameter monitoring method further comprises:
extracting first movement information which is caused by the movement that affects the vital sign of the measured object, when extracting the first vital sign information of the measured object from the measurement signal of the one measurement unit; and
at least correcting the first vital sign information, according to the first movement information and the second movement information, so as to obtain the physiological parameter of the measured object;
preferably, at least correcting the first vital sign information, according to the first movement information and the second movement information, so as to obtain the physiological parameter of the measured object, comprises:
processing the first movement information and the second movement information to obtain final movement information; and correcting the first vital sign information by using the final movement information, so as to obtain the physiological parameter of the measured object; or
correcting the first vital sign information, by using one of the first movement information and the second movement information, to obtain first vital sign data; and correcting the first vital sign data, by using the other one of the first movement information and the second movement information, so as to obtain the physiological parameter of the measured object.

14. A physiological parameter monitoring method, **characterized in that**, comprising:
extracting, from a measurement signal of one measurement unit, first vital sign information of a measured object and first movement information, which is caused by a movement that affects a vital sign of the measured object;
extracting, from a measurement signal of another measurement unit, second vital sign information of the measured object and second movement information, which is caused by the movement that affects the vital sign of the measured object; and
correcting at least one of the first vital sign information and the second vital sign information, according to at least one of the first movement information and the second movement information, so as to obtain a physiological parameter of the measured object.

15. The physiological parameter monitoring method according to claim 14, **characterized in that**:
correcting at least one of the first vital sign information and the second vital sign information, according to at least one of the first movement information and the second movement information, so as to obtain a physiological parameter of the measured object, comprises: analyzing the first vital sign information and the second vital sign information to obtain second vital sign data through an information quantification comparison; processing the first movement information and the second movement information to obtain final movement information; and correcting the second vital sign data by using the final movement information, so as to obtain the physiological parameter of the measured object; or
correcting at least one of the first vital sign information and the second vital sign information, according to at least one of the first movement information and the second movement information, so as to obtain a physiological parameter of the measured object, comprises: analyzing the first vital sign information and the second vital sign information to obtain second vital sign data through an information quantification comparison; correcting the second vital sign data, by using one of the first movement information and the second movement information, to obtain third vital sign data; correcting the third vital sign data, by using the other one of the first movement information and the second movement information, to obtain fourth vital sign data; and obtaining the physiological parameter of the measured object according to the third vital sign data or the fourth vital sign data; or
correcting at least one of the first vital sign information and the second vital sign information, according to at least one of the first movement information and the second movement information, so as to obtain a physiological parameter of the measured object, comprises: correcting the first vital sign information by using the first movement information to obtain fifth vital sign data; correcting the second vital sign information by using the second movement information to obtain sixth vital sign data; and analyzing the fifth vital sign data and the sixth vital sign data through a numerical quantification comparison, so as to obtain the physiological parameter of the measured object; or
correcting at least one of the first vital sign information and the second vital sign information, according to at least one of the first movement information and the second movement information, so as to obtain a physiological parameter of the measured object, comprises: correcting one of the first vital sign information and the second vital sign information, by using one of the first movement information and the second movement information, to obtain seventh vital sign data; and analyzing uncorrected vital sign information in the first vital sign information and the second vital sign information with the seventh vital sign data, so as to obtain the physiological parameter of the measured object through a numerical quantification comparison.
